(19) Europäisches Patentamt
European Patent Office
Office européen des brevets

(11) Veröffentlichungsnummer: **0 000 232**
**B1**

(12) # EUROPÄISCHE PATENTSCHRIFT

(45) Veröffentlichungstag der Patentschrift: **09.12.81**

(51) Int. Cl.³: **C 07 D 233/06**

(21) Anmeldenummer: **78200062.4**

(22) Anmeldetag: **27.06.78**

(54) **Verfahren zur Herstellung von blockierten Polyisocyanaten und danach erhältliche blockierte Polyisocyanate.**

(30) Priorität: **01.07.77 DE 2729704**

(43) Veröffentlichungstag der Anmeldung: **10.01.79 Patentblatt 79/1**

(45) Bekanntmachung des Hinweises auf die Patenterteilung: **09.12.81 Patentblatt 81/49**

(84) Benannte Vertragsstaaten: **BE CH DE FR GB LU NL**

(56) Entgegenhaltungen:
**Entgegenhaltungen**
**FR-A-1 466 282**

(73) Patentinhaber: **CHEMISCHE WERKE HÜLS AG, Postfach 1320, D-4370 Marl 1 (DE)**

(72) Erfinder: **Gras, Rainer, Dr., An der Ziegelei 91, D-4690 Herne 2 (DE)**
Erfinder: **Obendorf, Johann, Dr., Pastorsbusch 40, D-4270 Dorsten (DE)**
Erfinder: **Wolf, Elmar, Dr., Am Böckenbusch 3a, D-4690 Herne 2 (DE)**

(74) Vertreter: **Steil, Hanna, Dipl.-Chem. et al, RSP PATENTE-PB 40 Holsterhauser Strasse 160 Postfach 2840, D-4690 Herne 2 (DE)**

EP 0 000 232 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Verfahren zur Herstellung von blockierten Polyisocyanaten und danach erhältliche blockierte Polyisocyanate

Beschreibung und Beispiele

Gegenstand der vorliegenden Erfindung sind ein Verfahren zur Herstellung von neuen Verbindungen mit hohen Gehalt an latenten Isocyanatgruppen sowie die nach diesem Verfahren erhältlichen Verbindungen sowie ihre Verwendung.

Die Herstellung von verkappten Isocyanaten, auch Isocyanatabspalter genannt, ist bekannt und wird im Houben-Weyl, Methoden der organischen Chemie XIV/2. S. 61–70, beschrieben. Als Blockierungsmittel sind tertiäre Alkohole, Phenole, Acetessigester, Malonsäureester, Acetylaceton, Phthalimid, Imidazol, Chlorwasserstoff, Cyanwasserstoff und ε-Caprolactam bekannt. In der Praxis wird die Anwendung von Monophenolen und ε-Caprolactam für diesen Einsatzzweck bevorzugt.

Diese verkappten Isocyanate besitzen die Eigenschaft, bei erhöhter Temperatur wie Isocyanate zu reagieren. Die Abspaltung erfolgt umso leichter, je saurer das H-Atom der Maskierungsgruppe ist. Derartige blockierte Isocyanate werden in der DE-A-21 66 423 beschrieben. Auch sind endständige blockierte Isocyanate, die zusätzlich noch Uretdiongruppen enthalten, in der DE-A-25 02 934 beschrieben worden.

Der bedeutendste Nachteil bei der Verwendung dieser Blockierungsmittel ist bei einer Reihe von Einsatzzwecken die relativ hohe Abspalttemperatur, die für die meisten Phenole bei aliphatischen Polyisocyanaten bei mindestens 190 °C und bei aromatischen Polyisocyanaten ca. 30 °C niedriger liegt. Aus diesem Grunde ist in der Vergangenheit vorgeschlagen worden, Thiophenole zu verwenden, für die diese Temperatur viel niedriger ist. Der Nachteil der Thiophenole besteht jedoch in ihrem aussergewöhnlich unangenehmen Geruch.

Es wurde auch vorgeschlagen, 1–2 Prozent eines Katalysators hinzuzufügen, mittels welcher die Abspalttemperatur bis auf 125–140 °C reduziert werden könnte. Auf diese Weise wird jedoch die Gebrauchsdauer des ungehärteten Beschichtungsmaterials ungünstiger beeinflusst. Die Aufspalttemperatur von aliphatischen ε-Caprolactam-blockierten Polyisocyanaten liegt im allgemeinen bei ca. 180 °–190 °C.

Überraschenderweise wurde nunmehr gefunden, dass es durchaus möglich ist, auf einfache Weise blockierte Polyisocyanate herzustellen, die bei einer um mindestens 20 °–30 °C niedrigeren Temperatur deblockieren als die mit den herkömmlichen Blockierungsmitteln verkappten Isocyanaten, wenn man das Verfahren zur Herstellung von blockierten Polyisocyanaten durch Umsetzung von Polyisocyanaten mit N-haltigen cyclischen Verbindungen so durchführt, dass man als N-haltige cyclische Verbindungen cyclische Amidine der allgemeinen Formel

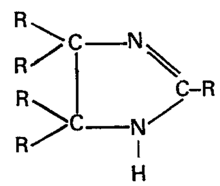

einsetzt, worin R gleiche oder verschiedene Substituenten aus der Gruppe Wasserstoff, Alkyl-, Cycloalkyl-, Aralkyl- und Arylrest sein können und die Umsetzung bei Temperaturen von 0–150 °C, vorzugsweise 80–120 °C erfolgt.

Die Polyisocyanate und die cyclischen Amidine werden in solchen Mengen eingesetzt, dass auf eine Isocyanatgruppe 0,5–1,1 vorzugsweise 0,8–1,0 Mol cyclisches Amidin kommen.

Die Umsetzung kann sowohl in Lösungsmitteln, in der Schmelze als auch in im Überschuss vorgelegtem Polyisocyanat durchgeführt werden.

Als Ausgangsverbindungen, die zur Blockierung mit den cyclischen Amidinen eingesetzt werden können, eignen sich beispielsweise Polyisocyanate, insbesondere Diisocyanate, wie aliphatische, cycloaliphatische, araliphatische, arylsubstituierte aliphatische und/oder aromatische Diisocyanate, wie sie beispielsweise in Houben-Weyl, Methoden der organischen Chemie, Band 14/2, S. 61–70 und dem Artikel von W. Siefken in Justus Liebigs Annalen der Chemie <u>562</u>, 75–136, beschrieben werden, wie 1,2-Äthylendiisocyanat, 1,4-Tetramethylendiisocyanat, 1,6-Hexamethylendiisocyanat, 2,2,4- bzw. 2,4,4-Trimethyl-1,6-hexamethylendiisocyanat (TMDI), 1,12-Dodecandiisocyanat, ω,ω'-Diisocyanatodipropyläther, Cyclobutan-1,3-diisocyanat, Cyclohexan-1,3- und 1,4-diisocyanat, 3-Isocyanatomethyl-3,5,5-trimethylcyclohexylisocyanat, welches auch als Isophorondiisocyanat bezeichnet und mit IPDI abgekürzt wird, Decahydro-8-methyl-(1,4-methano-naphthalen-2 (oder 3) 5-ylendimethylen-diisocyanat, Decahydro-4,7-methano-indan-1 (oder 2) 5 (oder 6)-ylendimethylen-diisocyanat, Hexahydro-4-7-methanindan-1-(oder 2) 5 (oder 6)-ylen-diisocyanat, Hexahydro-1,3- bzw.- 1,4-phenylen-diisocyanat, 2,4- und 2,6-Hexahydro-toluylendiisocyant, Perhydro-2,4'- und/oder -4,4'-diphenylmethan-diisocyanat, ω,ω'-Diisocyanato-1,4-diäthylbenzol, 1,4-Phenylendiisocyanat, 4,4'-Diisocyanato-diphenyl, 4,4'-Diisocyanato-3,3'-dichlor-diphenyl, 4,4'-Diisocyanato-3,3'-dimethoxy-diphenyl, 4,4'-Diisocyanato-3,3'-dimethyl-diphenyl, 4,4'-Diisocyanato-3,3'-diphenyl-diphenyl, 4,4'-Diisocyanato-diphenylmethan, Naphthylen-1,5-diisocyanat, Toluylendiisocyanate, Toluylen-2,4- bzw. 2,6-diisocyanat, N,N'-(4,4'-Dimethyl-3,3'-diisocyanatodiphenyl)-uretdion, m-Xylylen-diisocyanat, aber auch die Triisocyanate wie 2,4,4'-Triisocyanato-diphenyläther, 4,4',4''-Triisocyanatotriphenylmethan, Tris-(4-isocyanatophenyl)-thiophosphat, sowie beliebige Gemische dieser Isomeren. Weitere geeignete Isocyanate werden in dem genannten Artikel in den Annalen auf Seite 122 f. beschrieben.

Besonders bevorzugt werden in der Regel die technisch leicht zugängigen aliphatischen, cycloaliphatischen oder aromatischen Diisocyanate und besonders das 3-Isocyanatomethyl-3,5,5-trimethyl-cyclohexylisocyanat und 2,4-Toluylendiisocyanat sowie deren isomere Gemische.

Neben den monomeren Polyisocyanaten können als Ausgangsstoffe für die Blockierung mit den nachstehend ausführlich beschriebenen Imidazolinen selbstverständlich auch die dimeren und trimeren Formen der Polyisocyanate, wie Uretdione und Isocyanurate, eingesetzt werden, die nach bekannten Methoden herstellbar sind.

Unter Polyisocyanaten im Sinne der vorliegenden Erfindung werden auch solche verstanden, die vor der Blockierung mit den Imidazolinen einer Umsetzung zur Molekülvergrösserung mit den in der Isocyanatchemie gebräuchlichen sogenannten Kettenverlängerungsmitteln, wie Wasser, Polyolen, Polyaminen u.a., unterworfen wurden, wobei das bi- oder trifunktionelle Kettenverlängerungsmittel, also solche mit gegenüber Isocyanatgruppen reaktionsfähigen Gruppen, wie Hydroxyl- und/oder Aminogruppen, tragende Verbindungen, in solchen Mengen verwendet wird, dass das resultierende neue Isocyanat im Durchschnitt mindestens 2 Isocyanatgruppen trägt. Bei Verwendung von Wasser als Kettenverlängerungsmittel resultieren Polyisocyanate mit einer oder mehreren Harnstoffgruppierungen.

Geeignete Polyole sind beispielsweise Diole und Triole, z.B. Äthylenglykol, Propylenglykole, wie 1,2- und 1,3-Propandiol, 2,2-Dimethylpropandiol-(1,3), Butandiole, wie Butandiol-(1,4), Hexandiole, z.B. Hexandiol-(1,6), 2,2,4-Trimethylhexandiol-(1,6), 2,4,4-Trimethylhexandiol-(1,6), Heptandiol-(1,7), Octadecen-9,10-diol-(1,12), Thiodiglykol, Octadecandiol-(1,18), 2,4-Dimethyl-2-propylheptandiol-(1,3) Buten- oder Butindiol-(1,4), Diäthylenglykol, Triäthylenglykol, trans- und cis-1,4-Cyclohexandimethanol, 1,4-Cyclohexandiole, Glycerin, Hexantriol-(1,2,6), 1,1,1-Trimethylolpropan, 1,1,1-Trimethyloläthan u.a. Es können auch Mischungen der vorgenannten Verbindungen verwendet werden.

Von den für die Kettenverlängerung, bzw. Molekülvergrösserung geeigneten Polyaminen sollen beispielsweise das Äthylendiamin 1,2, Propylendiamin-1,2 und -1,3, Butylendiamin-1,2, -1,3 und -1,4 sowie die Hexamethylendiamine genannt sein.

Die Umsetzung der Polyisocyanate vor der Blokkierung mit den genannten Kettenverlängerungsmitteln in den angegebenen Mengenverhältnissen kann bei Temperaturen im Bereich von 0–150 °C, vorzugsweise 80–120 °C durchgeführt werden.

Geeignete Imidazolin-Derivate im Sinne der vorliegenden Erfindung, die der früher beschriebenen allgemeinen Formel entsprechen, sind beispielsweise solche mit gegebenenfalls arylsubstituierten Alkylresten, mit gegebenenfalls alkylsubstituierten Arylresten, wie 2-Methylimidazolin, 2,4-Dimethylimidazolin, 2-Methyl-4-(n-butyl)-imidazolin, 2-Äthylimidazolin, 2-Äthyl-4-methyl-imidazolin, 2-Benzyl-imidazolin, 2-Phenyl-imidazolin, 2-Phenyl-4-(N-morpholinylmethyl)-imidazolin, 2-(o-Tolyl)-imidazolin, 2-(p-Tolyl)-imidazolin, u.a.m. Es können auch Gemische der Imidazolin-Derivate erfindungsgemäss eingesetzt werden. Dieses ist besonders dann zweckmässig, wenn blockierte Isocyanate mit niedrigen Schmelzpunkten bzw. -bereichen benötigt werden.

Die erfindungsgemäss einsetzbaren Imidazolin-Derivate können nach bekannten Verfahren aus gegebenenfalls substituierten geminalen Diaminen und aliphatischen oder aromatischen Mononitrilen in Gegenwart von elementarem Schwefel oder Sulfurylchlorid als Katalysator hergestellt werden.

Gemäss FR-A 14 66 282 ist es bereits bekannt, Δ-1,2-Imidazolin-N-carboxyalkyl- bzw. carboxycycloalkylamide durch Umsetzung von Δ-1,2-Imidazolinen und Monoisocyanaten für therapeutische Zwecke herzustellen. Nicht bekannt ist bisher die Blockierung von Diisocyanaten mit Δ-1,2-Imidazolinen.

Die Blockierung kann, wie bereits erwähnt, auch in Lösungsmitteln durchgeführt werden. Als Lösungsmittel für diese Reaktion kommen nur solche infrage, die mit den Polyisocyanaten nicht reagieren, beispielsweise Ketone, wie Aceton, Methyläthylketon, Methylisobutylketon, Cyclopentanon, Cyclohexanon u.a; Aromaten, wie Benzol, Toluol, Xylole, Chlorbenzol, Nitrobenzol u.a.; cyclische Äther, wie Tetrahydrofuran, Dioxan u.a.; Ester, wie Methylacetat, n-Butylacetat u.a.; aliphatische Chlorkohlenwasserstoffe, wie Chloroform, Tetrachlorkohlenstoff u.a.; sowie aprotische Lösungsmittel, wie Dimethylformamid, Dimethylacetamid, Dimethylsulfoxid usw.

Wenn das Blockierungsmittel im Verhältnis von $\geqq 1$ zur Anzahl der Isocyanatgruppen eingesetzt wird, werden die Reaktionsmischungen so lange bei den angegebenen Temperaturen gehalten, bis der NCO-Gehalt der Reaktionsmischung auf Werte unter 0,2 % NCO abgesunken ist, ansonsten bis zur Erreichung eines konstanten NCO-Wertes.

Ein weiterer Gegenstand der vorliegenden Erfindung ist noch die nachträgliche Umsetzung einer Gruppe von blockierten Polyisocyanaten, nämlich solche, bei denen cyclische Amidine in unterstöchiometrischen Mengen eingesetzt worden sind, d.h. das Verhältnis cyclisches Amidin zu Isocyanatgruppen war $< 1 : 1$, mit den gleichen Kettenverlängerungsmitteln, die bereits früher als Mittel zur Molekülvergrösserung beschrieben worden sind. Die Umsetzung erfolgt ebenfalls bei Temperaturen im Bereich von 0–150 °C, vorzugsweise 80–120 °C, jedoch unterhalb der Deblockierungstemperatur des blockierten Polyisocyanats. Durch diese Verfahrensvariante lässt sich die Produktpalette der blockierten Polyisocyanate in sehr weiten Grenzen den praktischen Erfordernissen anpassen. Diese Verfahrensvariante ist besonders von Interesse für Polyisocyanate mit unterschiedlich reaktiven NCO-Gruppen, d.h. asymmetrischen Polyisocyanaten.

So lassen sich durch Wechsel der Reihenfolge Adduktbildung/Blockierung blockierte Polyisocyanate mit unterschiedlicher Reaktivität, Schmelzbereich und Struktur erhalten.

Gegenstand der Erfindung sind weiter auch die nach den beschriebenen Verfahren erhältlichen Addukte. Es handelt sich dabei im allgemeinen um Verbindungen des Molekulargewichtsbereichs von 300–2 500, vorzugsweise von 300–1 000. Die Verfahrensprodukte besitzen einen Schmelzbereich im Temperaturbereich von 30–220 °C, vorzugsweise 80–160 °C. Die mit den cyclischen Amidinen blockierten Polyisocyanate sind weiter durch einen Gehalt an endständig in blockierter Form vorliegenden Isocyanatgruppen (berechnet als NCO) von 4–25 Gew.-%, vorzugsweise 10–21 Gew.-%, charakterisiert.

Vorzugsweise handelt es sich um blockierte Polyisocyanate der folgenden Formeln:

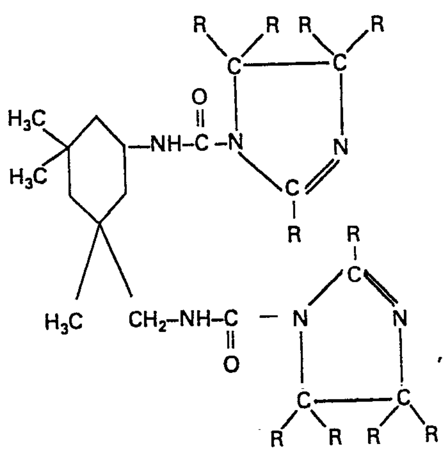

, in der

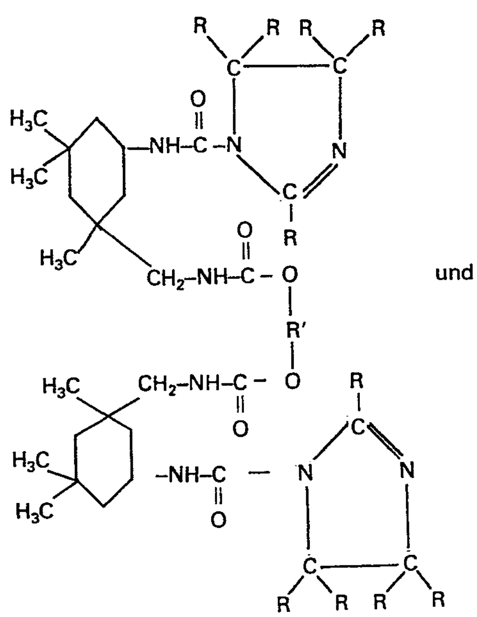

und

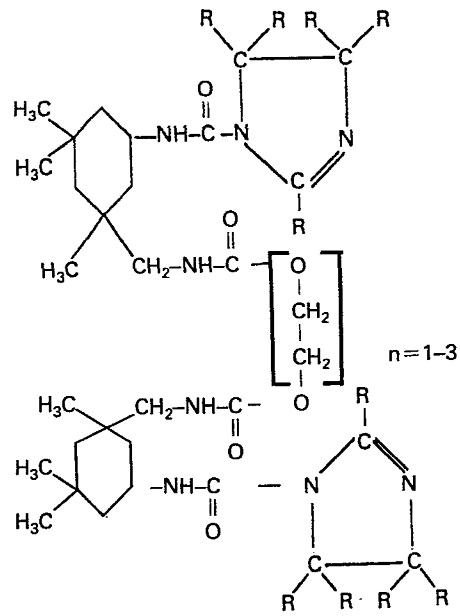

worin R gleiche oder verschiedene Substituenten aus der Gruppe Wasserstoff, Alkyl, Cycloalkyl, Aralkyl bedeutet und R' der zweiwertige organische Rest einer Verbindung mit 2 OH-Gruppen ist.

Die Verbindungen der vorstehend beschriebenen Erfindung eignen sich besonders wegen ihrer Schmelzpunkte bei gleichzeitig höheren Molekulargewichten als Katalysatoren für die anionische Polymerisation von ε-Caprolactam. Die Verbindungen können auch zur Herstellung von Drahtlacken verwendet werden.

Das Verfahren der vorliegenden Erfindung wird durch die nachstehenden Beispiele illustriert:

Beispiel 1

Zu einer Mischung aus 222 Gew.-T. Isophorondiisocyanat (IPDI) und 300 Gew.-T. wasserfreiem Aceton wurden bei Raumtemperatur langsam 292 Gew.-T. 2-Phenylimidazolin, die in 500 Gew.-T. wasserfreiem Aceton gelöst waren, zugetropft. Nach Beendigung der 2-Phenylimidazolinzugabe wurde eine Stunde bei 50 °C erhitzt. Das Aceton wurde abdestilliert. Die letzten Reste an Aceton wurden durch Trocknung des Reaktionsproduktes bei 60 °C im Vakuumtrockenschrank entfernt. Das mit 2-Phenylimidazolin blockierte IPDI stellt ein weisses Pulver mit einem Schmelzbereich von 98–106 °C dar, einer Glasumwandlungstemperatur (DTA) von 63–80 °C und besitzt eine Aufspalttemperatur von ca. 120 °C. Das IR-Spektrum dieses blockierten Polyisocyanates zeigt Abb. 1.

| | | | |
|---|---|---|---|
| berechnet | C: 70,07%; | H: 7,39%; | N: 16,34%; |
| gefunden | C: 69,91%; | H: 7,18%; | N: 16,25%. |

Beispiel 2

Zu einer Schmelze von 320 Gew.-T. 2-Phenyl-4-methylimidazolin wurden 222 Gew.-T. IPDI so zugetropft, dass die Temperatur im Reaktionskolben nicht über 120 °C stieg. Zur Vervollständigung der Reaktion wurde die Reaktionsmischung 3 h bei 120 °C gehalten. Diese Bedingungen reichten für

eine nahezu vollständige Umsetzung (NCO-Gehalt des Reaktionsproduktes 0,2 Gew.-%). Das Reaktionsprodukt ist ein weissses kristallines Pulver mit einem Schmelzbereich von 95–98 °C, einer Glasumwandlungstemperatur (DTA) von 65–85 °C und besitzt eine Aufspalttemperatur von ca. 140 °C. Das IR-Spektrum des blockierten Polyisocyanates zeigt Abb. 2.

berechnet C: 70,85%; H: 7,75%; N: 15,5%;
gefunden C: 70,69%; H: 7,53%; N: 15,36%.

Beispiel 3

Zu 222 Gew.-T. IPDI wurden bei 80 °C 196 Gew.-T. 2,4-Dimethylimidazolin so zugetropft, dass die Temperatur nicht über 90 °C stieg. Nach beendeter 2,4-Dimethyl-imidazolin-Zugabe wurde noch eine weitere Stunde bei 100 °C erhitzt. Das Reaktionsprodukt ist ein farbloses Pulver mit einem Schmelzbereich von 104–110 °C, einer Glasumwandlungstemperatur (DTA) von 75–90 °C und besitzt eine Aufspalttemperatur von ca. 160 °C. Das IR-Spektrum des blockierten Polyisocyanates zeigt Abb. 3.

berechnet C: 63,16%; H: 9,09%; N: 20,10%;
gefunden C: 62,98%; H: 8,91%; N: 20,21%.

Beispiel 4

a) Zu 444 Gew.-T. IPDI wurden bei 80 °C unter guter Rührung 106 Gew.-T. Diäthylenglykol langsam zugegeben. Nach erfolgter Diäthylenglykolzugabe wurde noch 2 h bei 80 °C erhitzt. Der NCO-Gehalt des IPDI/Diäthylenglykol-Gemisches betrug dann 15,1 Gew.-%.

b) Zu 556 Gew.-T. des in 4 a hergestellten Addukts aus 2 Molen IPDI und 1 Mol Diäthylenglykol wurden bei 120 °C portionsweise 292 Gew.-T. 2-Phenylimidazolin so zugegeben, dass die Temperatur nicht über 120 °C anstieg. Nach Beendigung der 2-Phenylimidazolinzugabe wurde das Reaktionsgemisch noch eine Stunde bei 120 °C erhitzt. Das Reaktionsprodukt ist ein blassgelbes Pulver mit einem Schmelzbereich von 100–106 °C, einer Glasumwandlungstemperatur (DTA) von 70–90 °C und besitzt eine Aufspalttemperatur von ca. 140 °C.

Das IR-Spektrum des blockierten Polyisocyanates zeigt Abb. 4.

Beispiel 5

Zu 556 Gew.T. des im Beispiel 4 a beschriebenen IPDI-Diäthylenglykol-Addukts werden bei 100 °C 320 Gew.-T. 2-Phenyl-4-Methylimidazolin so zugegeben, dass die Temperatur des Reaktionsgemisches nicht über 110 °C anstieg. Zur Vervollständigung der Reaktion wurde das Reaktionsgemisch noch 2 Stunden bei 110° erhitzt. Das Reaktionsprodukt ist ein weisses Pulver mit einem Schmelzbereich von 95–100 °C, einer Glasumwandlungstemperatur (DTA) von 60–85 °C und einer Aufspalttemperatur von ca. 150 °C. Im Reaktionsprodukt konnte NCO nicht mehr nachgewiesen werden.

Das IR-Spektrum des blockierten Polyisocyanates zeigt Abb. 5.

Beispiel 6

Zu 556 Gew.-T. des im Beispiel 4 a beschriebenen Addukts aus 2 Molen IPDI und 1 Mol Diäthylenglykol wurden bei 100 °C 196 Gew.-T. 2,4-Dimethylimidazolin so zugetropft, dass die Temperatur nicht über 110 °C anstieg. Nach erfolgter 2,4-Dimethylimidazolinzugabe wurde das Reaktionsgemisch noch weitere 2 Stunden bei 110 °C erhitzt. In dem so hergestellten Reaktionsprodukt konnte kein NCO mehr nachgewiesen werden. Das Reaktionsprodukt ist ein farbloses Produkt mit einem Schmelzbereich von 100–107 °C, einer Glasumwandlungstemperatur (DTA) von 60–95 °C und einer Aufspalttemperatur von ca. 170 °C.

Das IR-Spektrum des blockierten Polyisocyanates zeigt Abb. 6.

Beispiel 7

Zu einer Schmelze von 168 Gew.-T. 2-Methylimidazolin wurden 222 Gew.-T. IPDI so zugetropft, dass die Temperatur im Reaktionskolben nicht über 130 °C anstieg. Zur Vervollständigung der Reaktion wurde die Reaktionsmischung 3 h bei 130 °C gehalten.

Diese Bedingungen reichten für eine nahezu vollständige Umsetzung aus (NCO-Gehalt des Reaktionsproduktes 0,2 Gew.-%). Das Reaktionsprodukt stellt ein weisses kristallines Pulver mit einem Schmelzbereich von 107–110 °C dar, einem Glasumwandlungspunkt (DTA) von 82–89 °C und einer Aufspalttemperatur von ca. 150 °C.

Beispiel 8

Zu 556 Gew.-T. des in Beispiel 4 a beschriebenen Addukts aus 2 Molen IPDI und 1 Mol Diäthylenglykol wurden bei 120 °C 168 Gew.-T. 2-Methylimidazolin so zugegeben, dass die Temperatur des Reaktionsgemisches nicht über 110 °C stieg. Zur Vervollständigung der Reaktion wurde das Reaktionsgemisch noch 2 h bei 110 °C erhitzt. Das Reaktionsgemisch stellt ein weisses Pulver mit einem Schmelzbereich von 109–112 °C dar, einem Glasumwandlungspunkt (DTA) von 83–91 °C und einer Aufspalttemperatur von 160 °C.

Beispiel 9

Zu einer Schmelze von 210 Gew.-T. 2,4,4-(2,2,4)-Trimethylhexamethylendiisocyanat (1 : 1) wurden 292 Gew.-T. 2-Phenylimidazolin so zugetropft, dass die Temperatur im Reaktionskolben nicht über 120 °C stieg. Zur Vervollständigung der Reaktion wurde die Reaktionsmischung 3 h bei 120 °C gehalten. Diese Bedingungen reichten für eine nahezu vollständige Umsetzung (NCO-Gehalt des Reaktionsproduktes 0,1 Gew.-%). Das Reaktionsprodukt ist ein weisses kristallines Pulver mit einem Schmelzbereich von 64–76 °C, einer Glasumwandlungstemperatur (DTA) von 57–72 °C und besitzt eine Aufspalttemperatur von ca. 140 °C.

Das IR-Spektrum des blockierten Polyisocyanates zeigt Abb. 7.

berechnet C: 69,32 %; H: 7,57 %; N: 16,73 %;
gefunden C: 69,44 %; H: 7,43 %; N: 16,65 %.

Beispiel 10

Zu 210 Gew.-T. 2,4,4-(2,2,4)-Trimethylhexamethylendiisocyanat (1 : 1) wurden bei 80 °C 196 Gew.-T. 2,4-Dimethylimidazolin so zugetropft, dass die Temperatur nicht über 90 °C stieg. Nach beendeter 2,4-Dimethylimidazolinzugabe wurde noch eine weitere Stunde bei 100 °C erhitzt. (NCO-Gehalt des Reaktionsproduktes 0,1 Gew.-%). Das Reaktionsprodukt ist ein farbloses Pulver mit einem Schmelzbereich von 85–105 °C, eine Glasumwandlungstemperatur (DTA) von 70–91 °C und besitzt eine Aufspalttemperatur von ca. 150 °C.

Das IR-Spektrum des blockierten Polyisocyanates zeigt Abb. 8.

berechnet C: 72,07 %; H: 9,36 % N: 20,69 %;
gefunden C: 72,17 %; H: 9,19 %; N: 20,51 %.

Beispiel 11

Zu einer Schmelze von 210 Gew.-T. 2,4,4-(2,2,4)-Trimethylhexamethylendiisocyanat (1 : 1) wurden 164 Gew.-T. 2-Methylimidazolin so zugetropft, dass die Temperatur im Reaktionskolben nicht über 130 °C anstieg. Zur Vervollständigung der Reaktion wurde die Reaktionsmischung 3 h bei 130 °C gehalten. Diese Bedingungen reichten für eine nahezu vollständige Umsetzung aus (NCO-Gehalt des Reaktionsproduktes 0,1 Gew.-%). Das Reaktionsprodukt stellt ein weisses kristallines Pulver mit einem Schmelzbereich von 65–78 °C dar, einem Glasumwandlungspunkt (DTA) von 59–76 °C und einer Aufspalttemperatur von ca. 150 °C.

Beispiel 12

Zu einer Schmelze von 174 Gew.-T. Toluylen-2,4-(2,6)-diisocyanat (aus 80 % 2,4- und 20 % 2,6-) wurden 292 Gew.-T. 2-Phenylimidazolin so zugetropft, dass die Temperatur im Reaktionskolben nicht über 140 °C stieg. Zur Vervollständigung der Reaktion wurde die Reaktionsmischung 3 h bei 140 °C gehalten. Diese Bedingungen reichten für eine nahezu vollständige Umsetzung (NCO-Gehalt des Reaktionsproduktes 0,2 Gew.-%). Das Reaktionsprodukt ist ein weisses kristallines Pulver mit einem Schmelzbereich von 90–103 °C, einer Glasumwandlungstemperatur (DTA) von 78–90 °C und besitzt eine Aufspalttemperatur von ca. 130 °C.

Das IR-Spektrum des blockierten Polyisocyanates zeigt Abb. 9.

berechnet C: 69,53 %; H: 5,58 %; N: 18,03 %
gefunden C: 69,41 %; H: 5,35 %; N: 18,17 %

Beispiel 13

Zu einer Mischung aus 174 Gew.-T. Toluylen-2,4-(2,6)-diisocyanat (aus 80 % 2,4- und 20 % 2,6-) und 300 Gew.-T. wasserfreiem Aceton wurden bei Raumtemperatur langsam 196 Gew.-T. 2,4-Dimethylimidazolin, die in 500 Gew.-T. wasserfreiem Aceton gelöst waren, zugetropft. Nach Beendigung der 2,4-Dimethylimidazolinzugabe wurde eine Stunde bei 50 °C erhitzt. Das Aceton wurde abdestilliert. Die letzten Reste an Aceton wurden durch Trocknung des Reaktionsproduktes bei 60 °C im Vakuumtrockenschrank entfernt (NCO-Gehalt des Reatkionsproduktes 0,1 Gew.-%). Das mit 2,4-Dimethylimidazolin blockierte Diisocyanat stellt ein weisses Pulver mit einem Schmelzbereich von 85–105 °C dar, einer Glasumwandlungstemperatur (DTA) von 70–91 °C und besitzt eine Aufspalttemperatur von ca. 150 °C.

Das IR-Spektrum dieses blockierten Polyisocyanates zeigt Abb. 10.

berechnet C: 61,62 %; H: 7,03 %; N: 20,70 %;
gefunden C: 61,46 %; H: 6,89 %; N: 20,61 %.

Beispiel 14

Zu einer Schmelze von 174 Gew.-T. Toluylen-2,4-(2,6)-diisocyanat (aus 80% 2,4- und 20% 2,6-) wurden 320 Gew.-T. 2-Phenyl-4-methylimidazolin so zugetropft, dass die Temperatur im Reaktionskolben nicht über 140 °C stieg. Zur Vervollständigung der Reaktion wurde die Reaktionsmischung 3 h bei 140 °C gehalten. Diese Bedingungen reichten für eine nahezu vollständige Umsetzung (NCO-Gehalt des Reaktionsproduktes 0,15 Gew.-%). Das Reaktionsprodukt ist ein weisses kristallines Pulver mit einem Schmelzbereich von 82–99 °C, einer Glasumwandlungstemperatur (DTA) von 68–89 °C und besitzt eine Aufspalttemperatur von ca. 130 °C.

Beispiel 15

a) Zu 420 Gew.-T. 2,4,4- bzw. 2,2,4-Trimethylhexamethylendiisocyanat (1 : 1) wurden bei 80 °C unter guter Rührung 106 Gew.-T. Diäthylenglykol langsam zugegeben. Nach erfolgter Diäthylenglykolzugabe wurde noch 2 h bei 80 °C erhitzt. Der NCO-Gehalt des 2,4,4-(2,2,4)-Trimethylhexamethylendiisocyanat/Diäthylenglykol-Addukts betrug dann 15,8 Gew.-%.

b) Zu 532 Gew.-T. des in 15 a hergestellten Addukts aus 2 Molen 2,4,4-(2,2,4)-Trimethylhexamethylendiisocyanat und 1 Mol Diäthylenglykol wurden bei 120 °C portionsweise 292 Gew.-T. 2-Phenylimidazolin so zugegeben, dass die Temperatur nicht über 120 °C anstieg. Nach Beendigung der 2-Phenylimidazolinzugabe wurde das Reaktionsgemisch noch eine Stunde bei 120 °C erhitzt. Das Reaktionsprodukt ist ein blassgelbes Pulver mit einem Schmelzbereich von 66–83 °C, einer Glasumwandlungstemperatur (DTA) von 59–75 °C und besitzt eine Aufspalttemperatur von ca. 150 °C.

Beispiel 16

Zu 532 Gew.-T. des in 15 a hergestellten Addukts aus 2 Molen 2,4,4- bzw. 2,2,4-Trimethylhexamethylendiisocyanat und 1 Mol Diäthylenglykol wurden bei 120 °C portionsweise 196 Gew.-T. 2,4-Dimethylimidazolin so zugegeben, dass die Temperatur nicht über 120 °C anstieg. Nach Beendigung der 2,4-Dimethylimidazolinzugabe wurde das Reaktionsgemisch noch eine Stunde bei 120 °C erhitzt. Das Reaktionsprodukt ist ein blassgelbes Pulver mit einem Schmelzbereich von 71–

82 °C einer Glasumwandlungstemperatur (DTA) von 61–81 °C, und besitzt eine Aufspalttemperatur von ca. 170 °C.

Beispiel 17

a) Zu 348 Gew.-T. Toluylen-2,4-(2,6)-diisocyanat (aus 80 % 2,4- und 20 % 2,6-) wurden bei 80 °C unter guter Rührung 106 Gew.-T. Diäthylenglykol langsam zugegeben. Nach erfolgter Diäthylenglykolzugabe wurde noch 2 h bei 80 °C erhitzt. Der NCO-Gehalt des Toluylen-2,4-(2,6)-diisocyanat/ Diäthylenglykol-Gemisches betrug dann 18,3 Gew.-%.

b) Zu 459 Gew.T. des in 17 a hergestellten Addukts aus 2 Molen Toluylen-2,4-(2,6)-diisocyanat (aus 80 % 2,4- und 20 % 2,6-) und 1 Mol Diäthylenglykol wurden bei 160 °C portionsweise 292 Gew.-T. 2-Phenylimidazolin so zugegeben, dass die Temperatur nicht über 160 °C anstieg. Nach Beendigung der 2-Phenylimidazolinzugabe wurde das Reaktionsgemisch noch eine Stunde bei 160 °C erhitzt (NCO-Gehalt des Reaktionsproduktes 0,15 Gew.-%). Das Reaktionsprodukt ist ein blassgelbes Pulver mit einem Schmelzbereich von 92–105 °C, einer Glasumwandlungstemperatur (DTA) von 79–92 °C und besitzt eine Aufspalttemperatur von ca. 140 °C.

Beispiel 18

Zu 459 Gew.-T. des in 17 a hergestellten Addukts aus 2 Molen Toluylen-2,4-(2,6)-diisocyanat (aus 80 % 2,4- und 20 % 2,6-) und 1 Mol Diäthylenglykol wurden bei 150 °C portionsweise 196 Gew.-T. 2,4-Dimethylimidazolin so zugegeben, dass die Temperatur nicht über 150 °C anstieg. Nach Beendigung der 2,4-Dimethylimidazolinzugabe wurde das Reaktionsgemisch noch eine Stunde bei 150 °C erhitzt (NCO-Gehalt des Reaktionsproduktes 0,1 Gew.-%). Das Reaktionsprodukt ist ein blassgelbes Pulver mit einem Schmelzbereich von 90—107 °C, einer Glasumwandlungstemperatur (DTA) von 73–95 °C und besitzt eine Aufspalttemperatur von ca. 160 °C.

**Patentansprüche**

1. Verfahren zur Herstellung von blockierten Polyisocyanaten durch Umsetzung von Polyisocyanaten mit N-haltigen cyclischen Verbindungen, dadurch gekennzeichnet, dass man als N-haltige cyclische Verbindungen cyclische Amidine der allgemeinen Formel

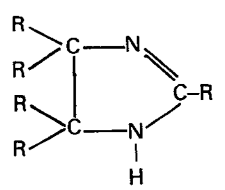

einsetzt, worin R gleiche oder verschiedene Substituenten aus der Gruppe Wasserstoff, Alkyl-, Cycloalkyl-, Aralkyl- und Arylrest sind und die Umsetzung bei Temperaturen von 0–150 °C, vorzugsweise 80–120 °C durchführt.

2. Verfahren nach Anspruch 1, dadurch gekennzeichnet, dass man die Polyisocyanate und die cyclischen Amidine in solchen Mengen einsetzt, dass auf eine Isocyanatgruppe 0,5–1,1, vorzugsweise 0,8–1,0 Mol cyclisches Amidin kommen.

3. Verfahren nach Anspruch 2, dadurch gekennzeichnet, dass man die blockierten Polyisocyanate, bei denen cyclische Amidine in unterstöchiometrischen Mengen eingesetzt wurden, anschliessend einer Umsetzung mit mehrwertigen Kettenverlängerungsmitteln bei Temperaturen im Bereich von 0–150 °C, jedoch unter der Deblockierungstemperatur, umsetzt und gegebenenfalls das überschüssige Kettenverlängerungsmittel entweder allein oder mit einem gegebenenfalls eingesetzten Lösungsmittel auf bekannte Weise entfernt.

4. Verfahren nach Anspruch 3, dadurch gekennzeichnet, dass man die Umsetzung bei Temperaturen im Bereich von 80–120 °C durchführt.

5. Verfahren nach den Ansprüchen 3 und 4, dadurch gekennzeichnet, dass das Kettenverlängerungsmittel Wasser ist.

6. Verfahren nach den Ansprüchen 3 und 4, dadurch gekennzeichnet, dass die Kettenverlängerungsmittel Polyole sind.

7. Verfahren nach den Ansprüchen 3 und 4, dadurch gekennzeichnet, dass die Kettenverlängerungsmittel Polyamine sind.

8. Blockierte Polyisocyanate, erhältlich nach den Ansprüchen 1–7.

9. Blockiertes Polyisocyanat der Formel

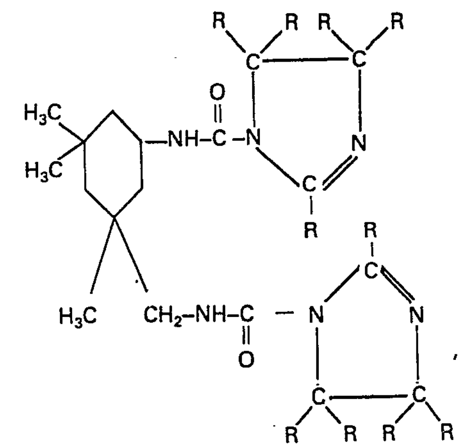

, in der

R die vorstehende Bedeutung hat.

10. Blockiertes Polyisocyanat der Formel

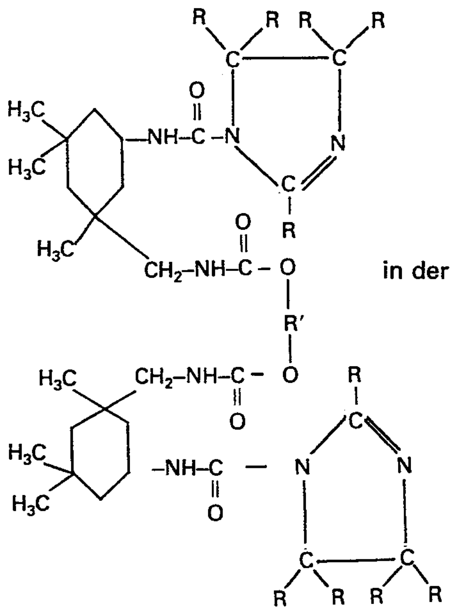

in der R die vorstehende Bedeutung hat und R' der zweiwertige organische Rest einer Verbindung mit 2 OH-Gruppen ist.

11. Blockierte Polyisocyanate der Formel

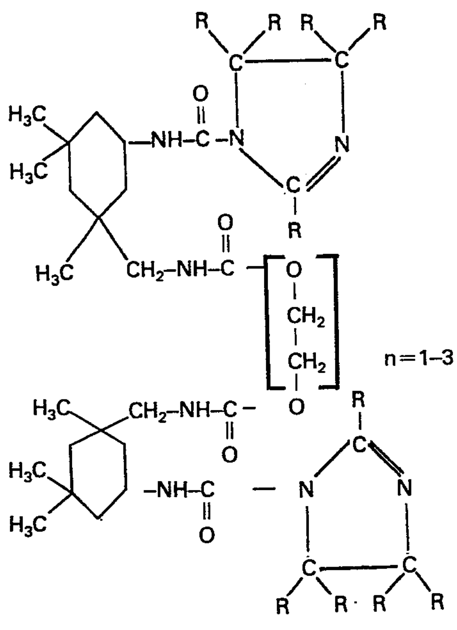

**Revendications**

1. Procédé de fabrication de polyisocyanates bloqués par réaction de polyisocyanates avec des composés cycliques azotés, caractérisé en ce qu'on utilise comme composés cycliques azotés des amidines cycliques de formule générale:

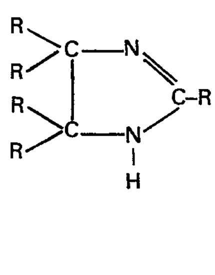

dans laquelle les R sont des substituants identiques ou différents du groupe hydrogène, radical alcoyle, cycloalcoyle, aralcoyle et aryle et en ce que la réaction est exécutée à des températures de 0–150 °C, de préférence de 80–120 °C.

2. Procédé selon la revendication 1, caractérisé en ce qu'on utilise les polyisocanates et les amidines cycliques en des quantités telles que pour un groupe isocyanate il y ait 0,5–1,1, de préférence 0,8–1,0 mole d'amidine cyclique.

3. Procédé selon la revendication 2, caractérisé en ce qu'ensuite on soumet les polyisocyanates bloqués, dans lesquels les amidines cycliques ont été utilisées en des quantités sub-stoechiométriques, à une réaction avec des agents d'allongement de chaîne polyvalents à des températures dans l'intervalle de 0–150 °C, toutefois au-dessous de la température de déblocage, et en ce qu'éventuellement on élimine l'agent d'allongement de chaîne en excès soit isolément ou avec un solvant éventuellement employé, de manière connue.

4. Procédé selon la revendication 3, caractérisé en ce qu'on exécute la réaction à des températures dans l'intervalle de 80–120 °C.

5. Procédé selon les revendications 3 et 4, caractérisé en ce que l'agent d'allongement de chaîne est de l'eau.

6. Procédé selon les revendications 3 et 4, caractérisé en ce que les agents d'allongement de chaîne sont des polyols.

7. Procédé selon les revendications 3 et 4, caractérisé en ce que les agents d'allongement de chaîne sont les polyamines.

8. Polyisocyanates bloqués, pouvant être obtenus selon les revendications 1 à 7.

9. Polyisocyanate bloqué de formule:

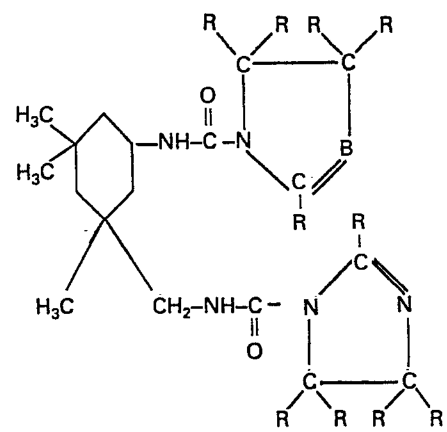

dans laquelle
R a la signification indiquée plus haut.

10. Polyisocyanate bloqué de formule:

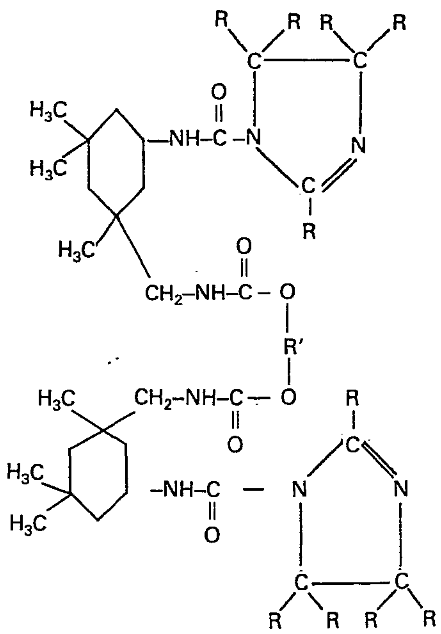

dans laquelle
R a la signification indiquée plus haut et R' est le radical organique bivalent d'un composé ayant 2 groupes OH.

11. Polyisocyanates bloqués de formule

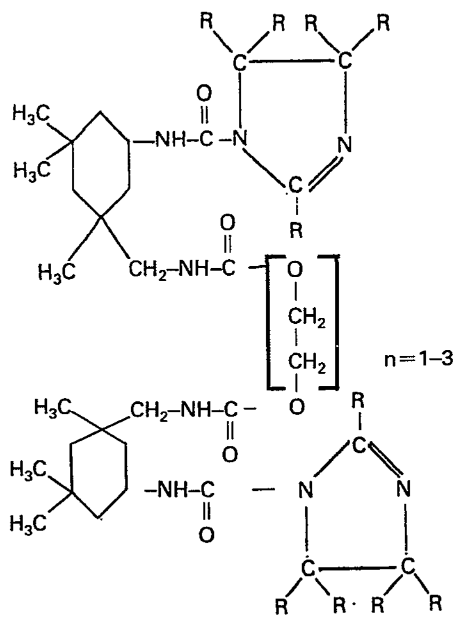

n=1–3

**Claims**

1. A method of producing blocked polyisocyanates by reacting polyisocyanates with cyclic nitrogenous compounds which comprises using cyclic amidines of the general formula

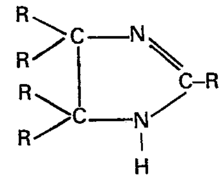

as the cyclic nitrogenous compounds, wherein each R may be the same or different substitute from the hydrogen, alkyl-, cycloalkyl-, aralkyl- and arylradical group and the reaction is carried out at temperatures of 0–150 °C, preferably 80–120 °C.

2. A method according to Claim 1, wherein the polyisocyanates and cyclic amidines are introduced in such quantities that 0,5–1,1, preferably 0,8–1,0 mole of cyclic amidine is combined with an isocyanate group.

3. A method according to Claim 2, wherein the blocked polyisocyanates to which cyclic amidines have been added in sub-stoichiometric quantities, are then reacted with polyvalent chain-lengthening agents at temperatures in the range of 0–150 °C, but below the deblocking temperature and any excess chain-lengthening agent is removed in a known manner either directly or with a solvent, if necessary.

4. A method according to Claim 3, wherein the reaction is carried out at temperatures in the range of 80–120 °C.

5. A method according to Claims 3 and 4, wherein the clain-lengthening agent is water.

6. A method according to Claims 3 and 4, wherein the chain-lengthening agents are polyols.

7. A method according to Claims 3 and 4, wherein the chain-lengthening agents are polyamines.

8. Blocked polyisocyanates according to Claims 1–7.

9. A blocked polyisocyanate of the formula

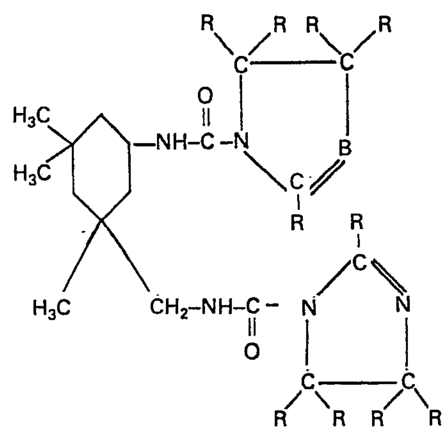

wherein R has the same meaning as before.

10. A blocked polyisocyanate of the formula

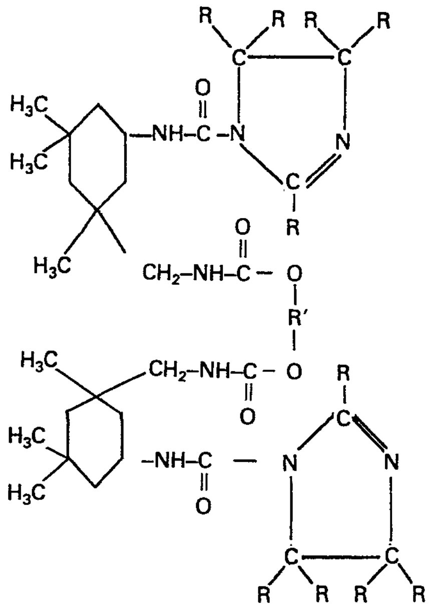

wherein R has the same meaning as before and R' is the bivalent organic radical of a compound with 2 OH-groups.

11. Blocked polyisocyanates of the formula

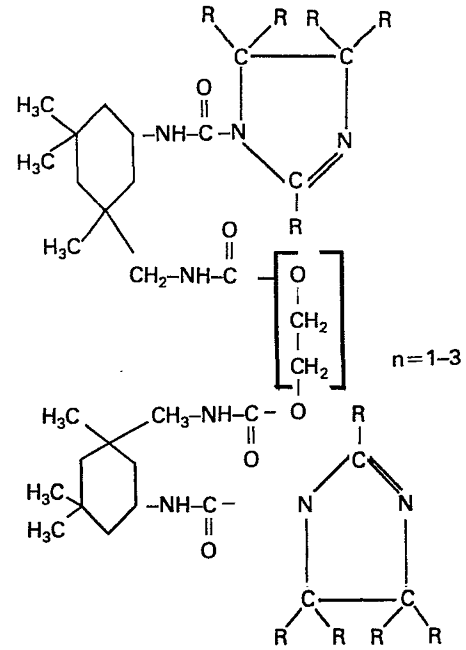

Abb. 1

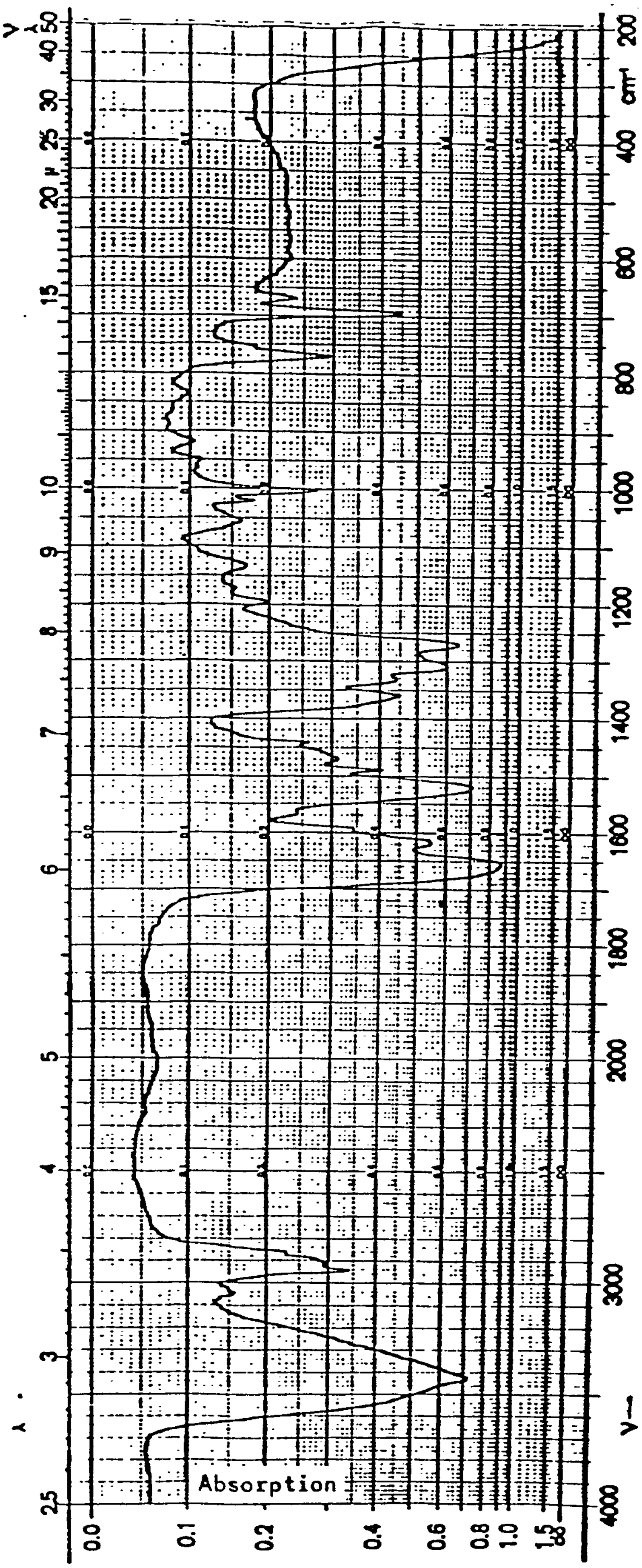
Absorption
cm⁻¹
0.0
0.1
0.2
0.4
0.6
0.8
1.0
1.5
∞
200
400
600
800
1000
1200
1400
1600
1800
2000
3000
4000

Abb. 2

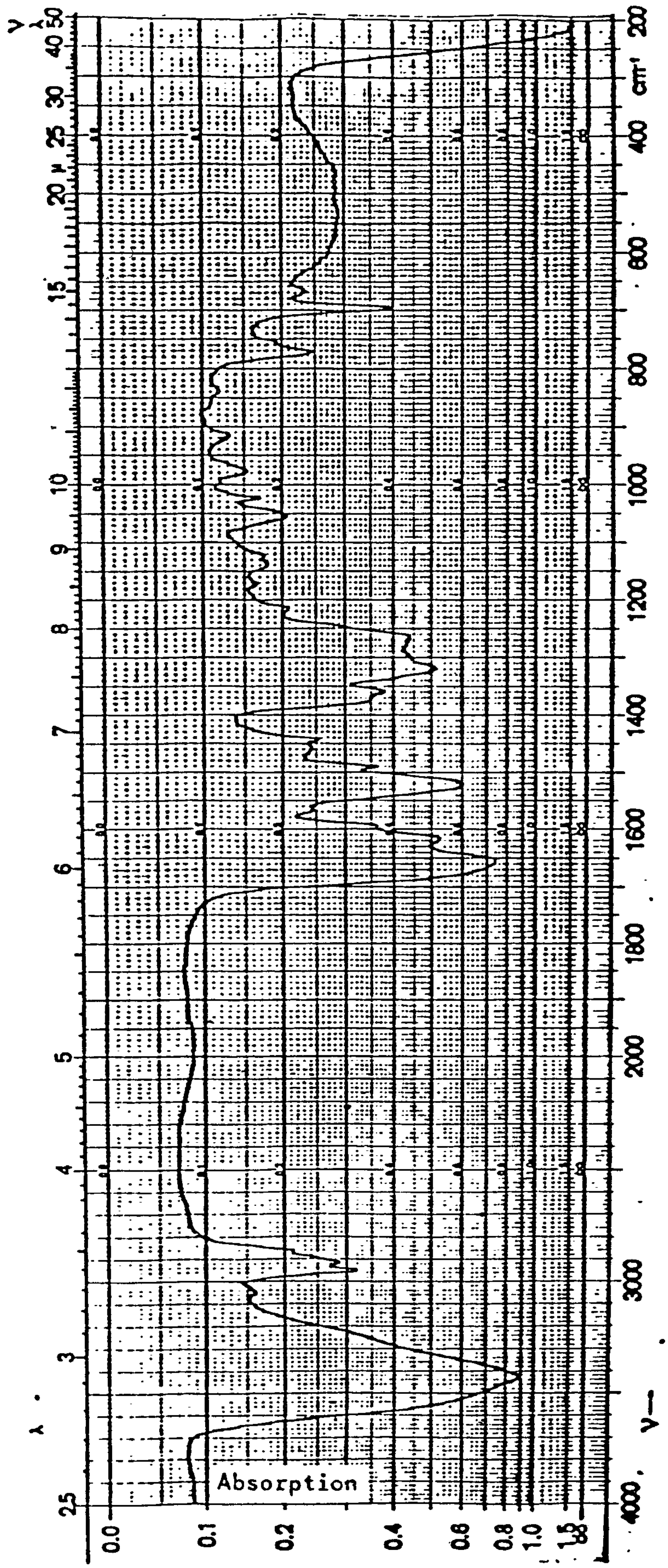
Absorption
cm⁻¹
4000
3000
2000
1800
1600
1400
1200
1000
800
600
400
200
0.0
0.1
0.2
0.4
0.6
0.8
1.0
1.5
∞

Abb. 3

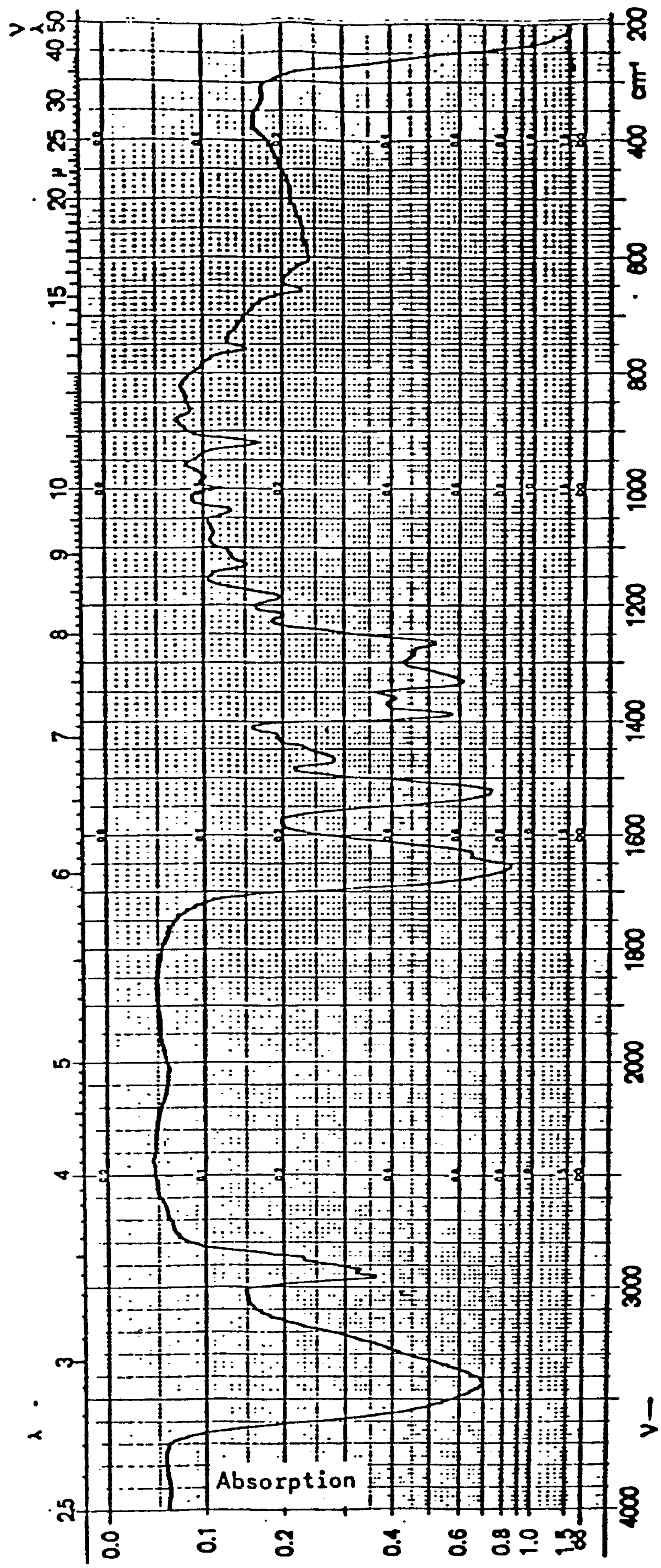

Abb. 4

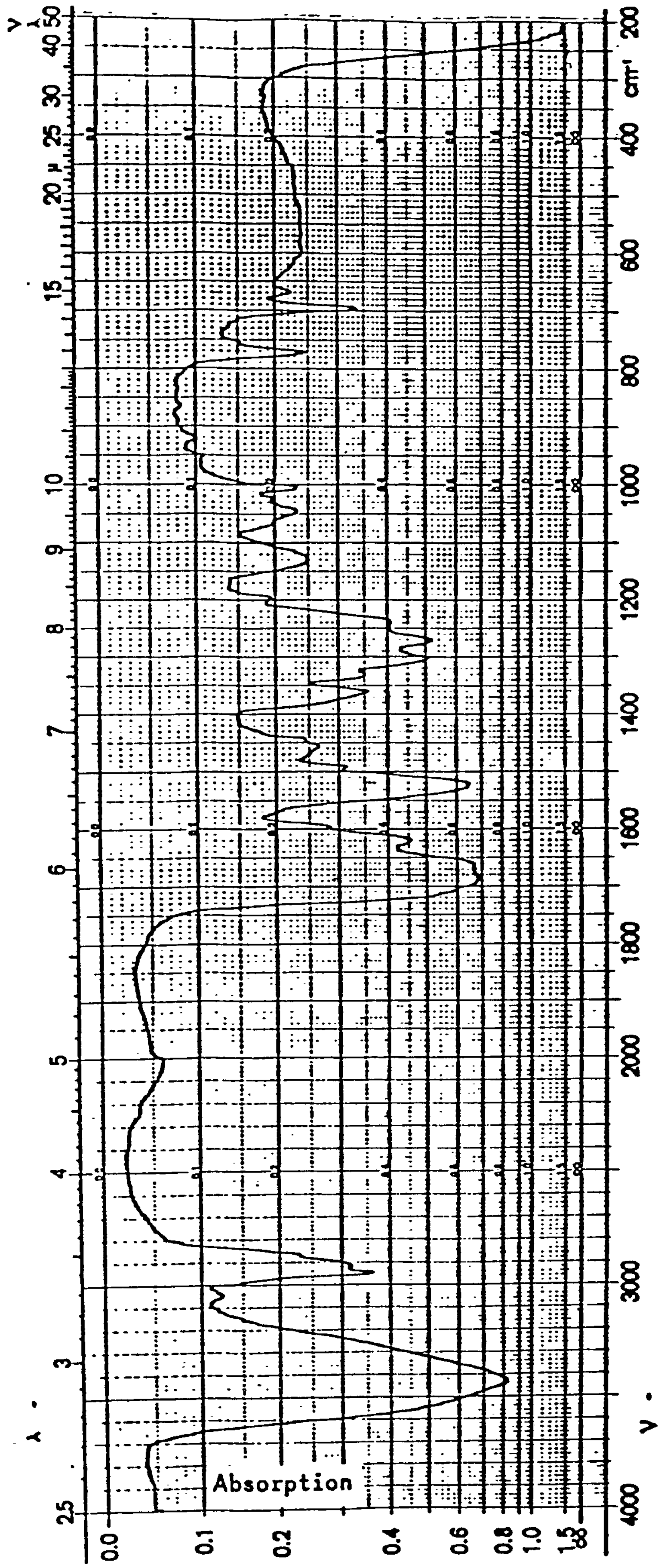

Abb: 5

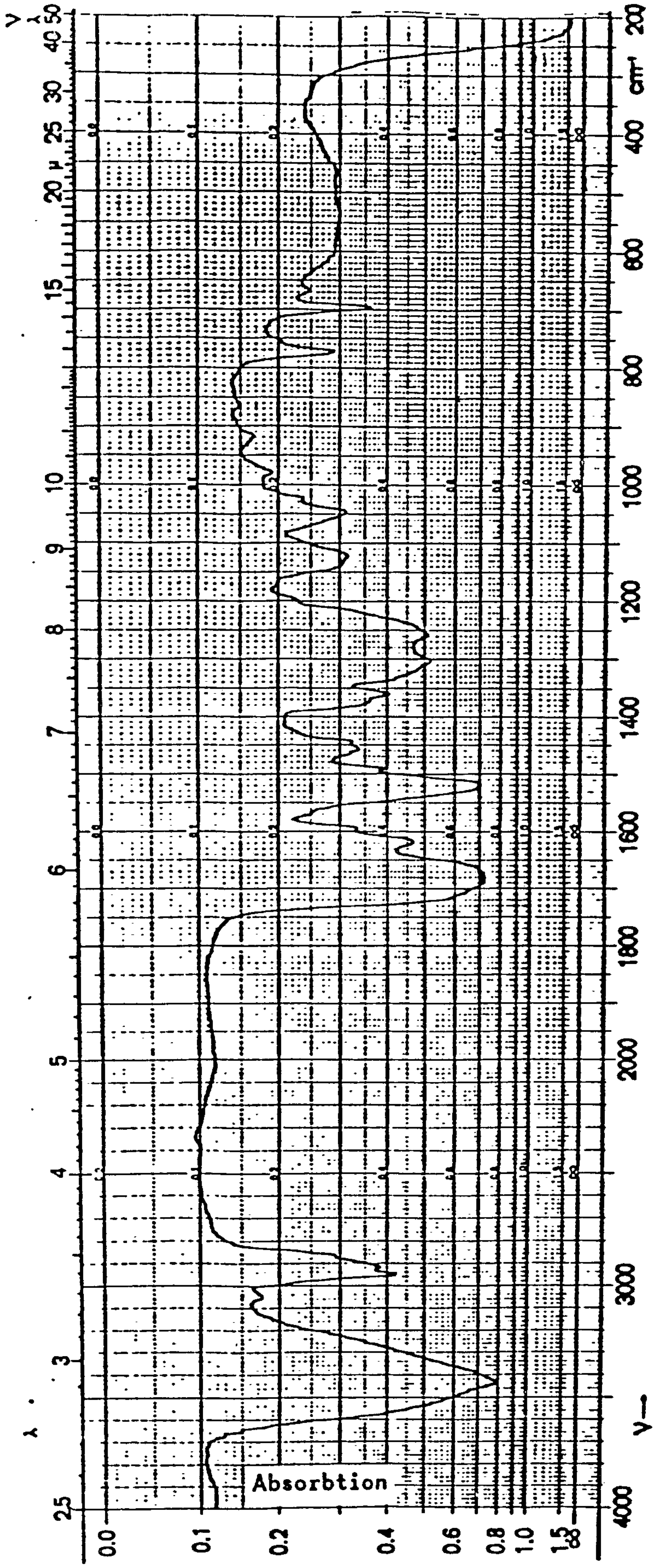
Absorbtion
cm⁻¹
0.0
0.1
0.2
0.4
0.6
0.8
1.0
1.5
∞
4000
3000
2000
1800
1600
1400
1200
1000
800
600
400
200
2,5
3
4
5
6
7
8
9
10
15
20 μ
25
30
40
50

Abb. 6

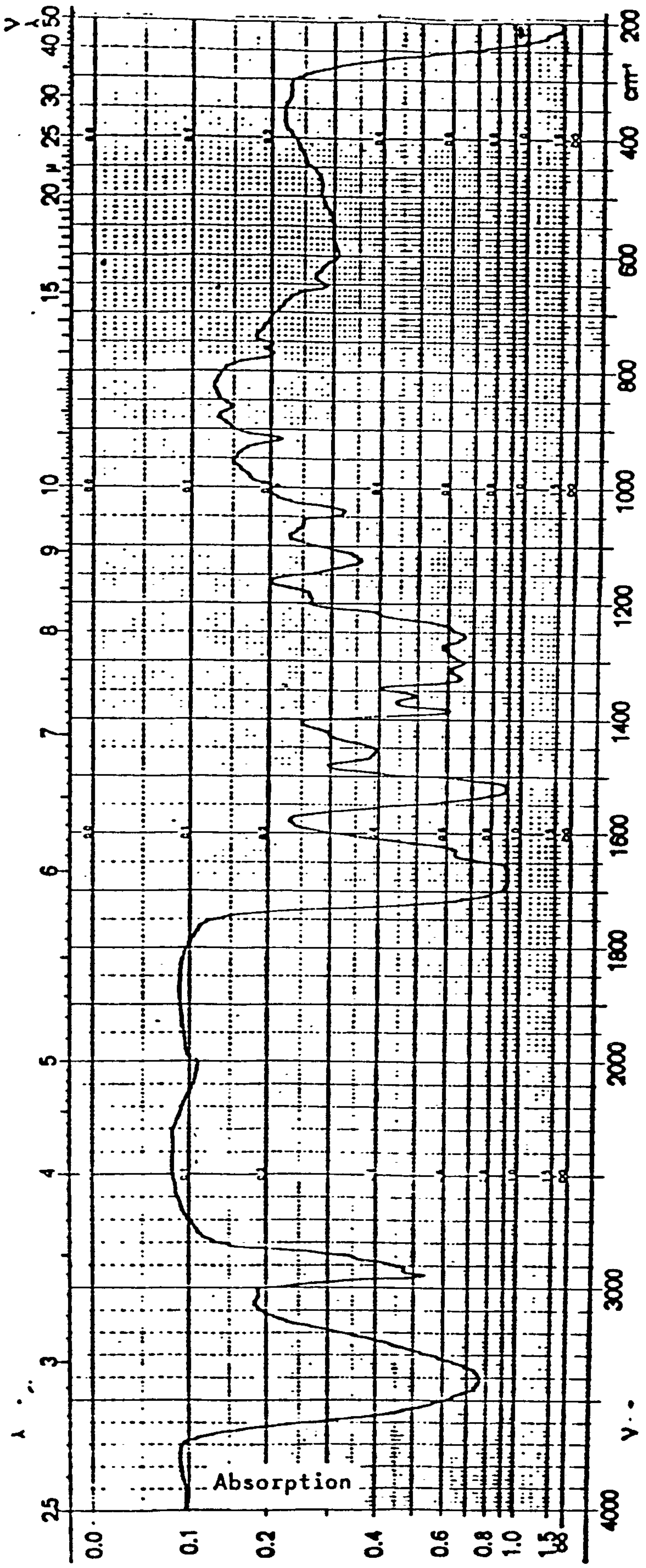
Absorption
cm⁻¹
200
400
600
800
1000
1200
1400
1600
1800
2000
3000
4000
0.0
0.1
0.2
0.4
0.6
0.8
1.0
1.5
∞
2.5
3
4
5
6
7
8
9
10
15
20 μ
25
30
40
50

Abb. 7

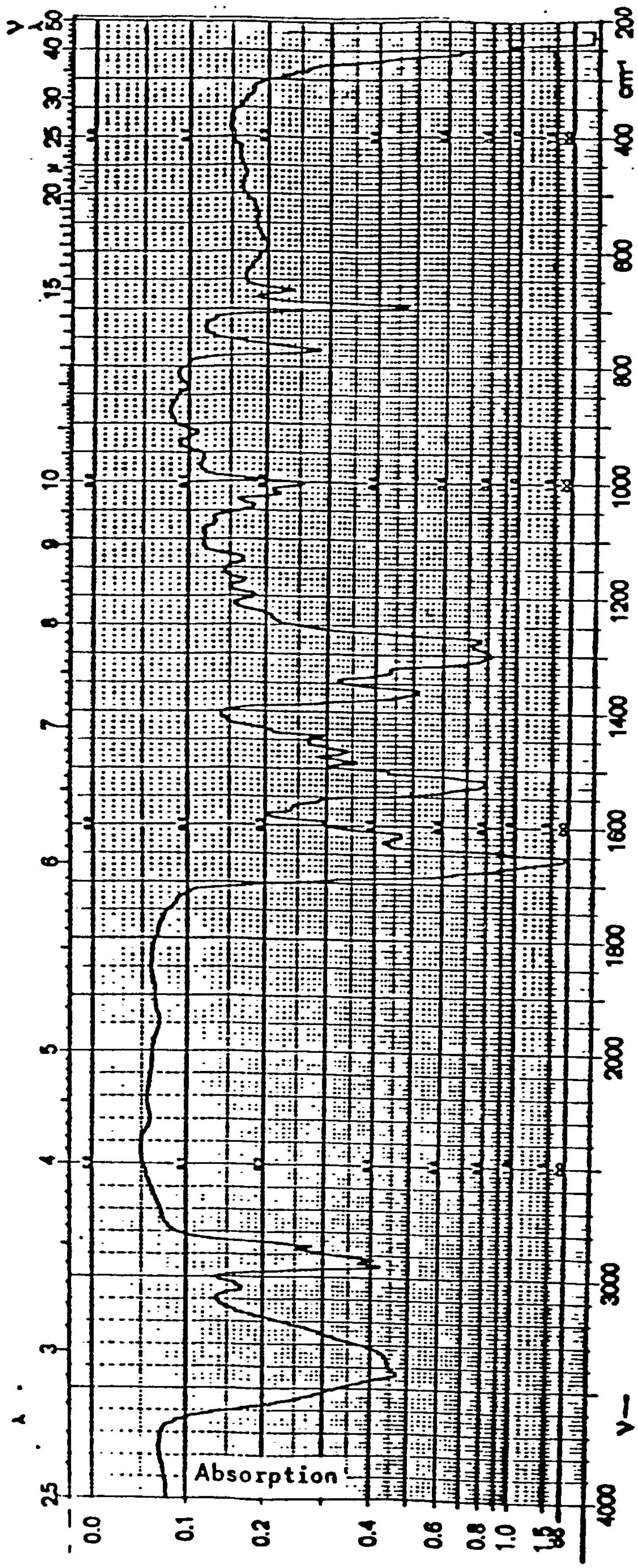
Absorption
cm⁻¹
4000
3000
2000
1800
1600
1400
1200
1000
800
600
400
200
0.0
0.1
0.2
0.4
0.6
0.8
1.0
1.5
2.5
3
4
5
6
7
8
9
10
15
20 μ
25
30
40
50

Abb. 8

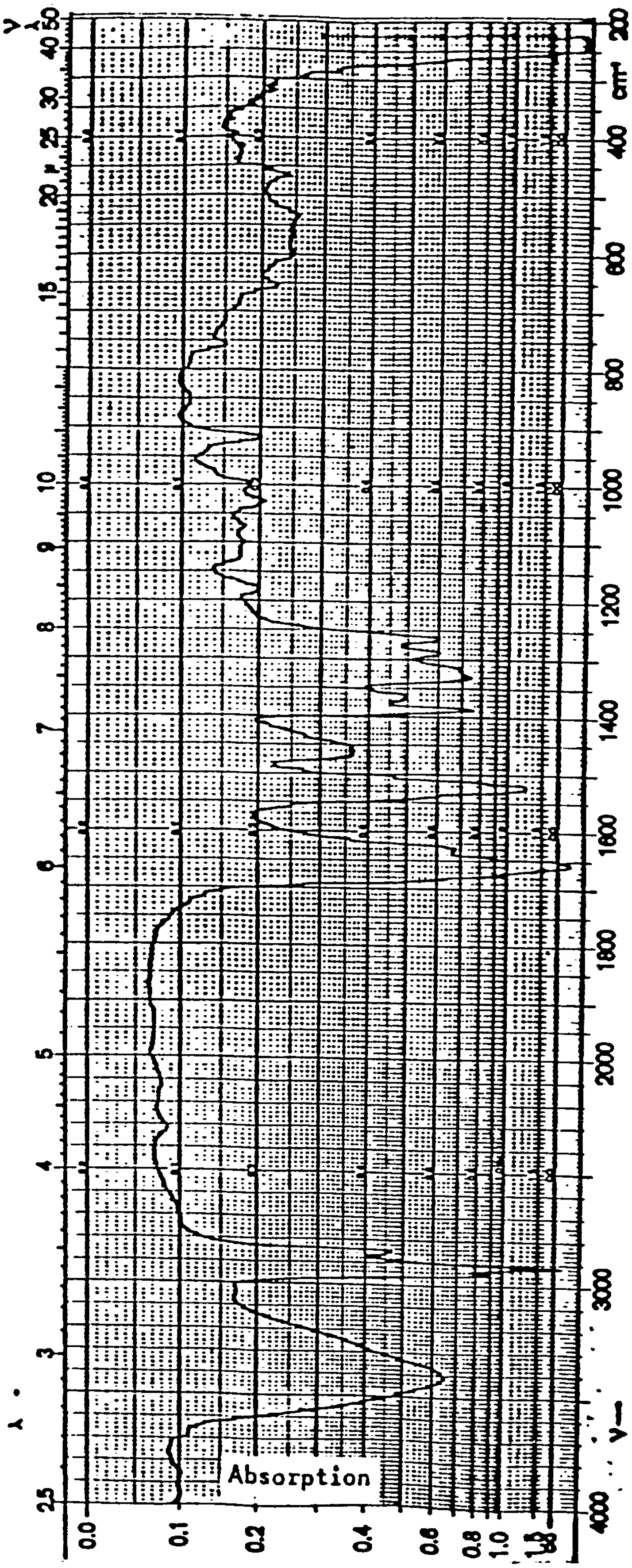

Abb. 9

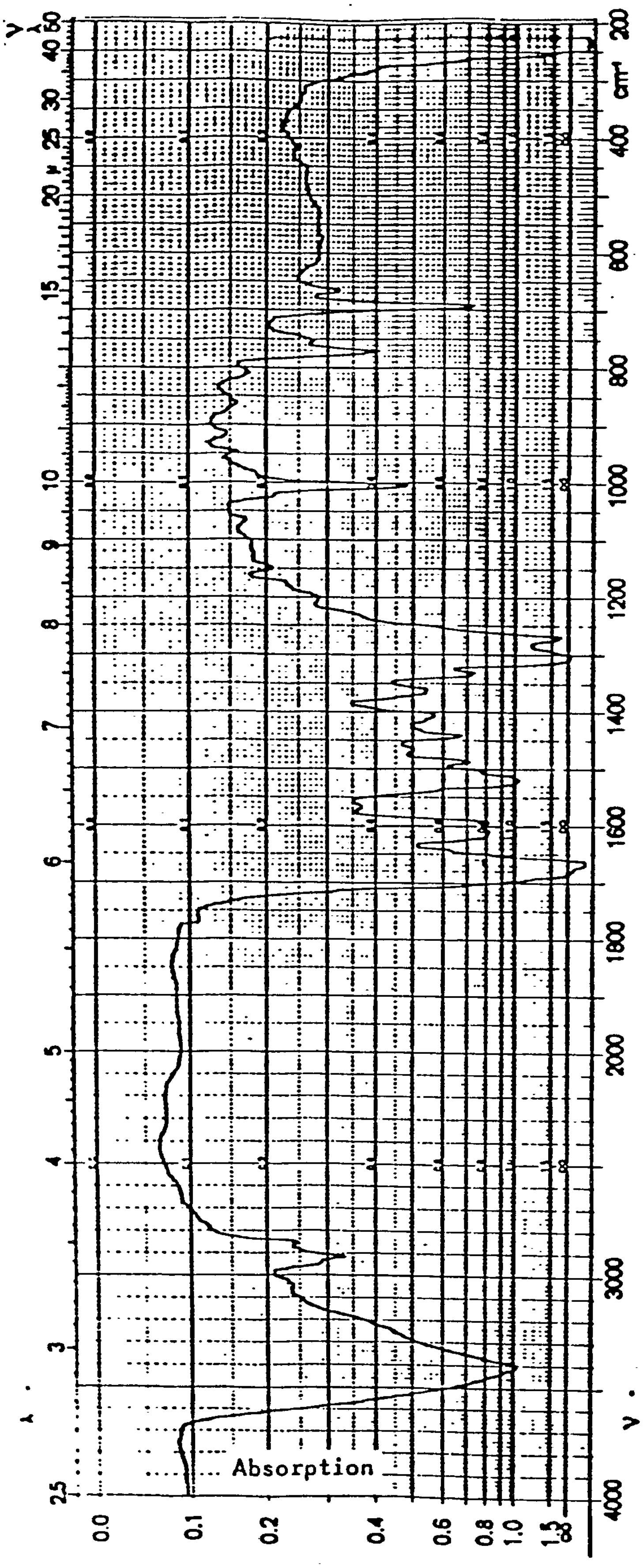
Absorption
cm⁻¹
0.0
0.1
0.2
0.4
0.6
0.8
1.0
1.5
∞
200
400
600
800
1000
1200
1400
1600
1800
2000
3000
4000
2.5
3
4
5
6
7
8
9
10
15
20 μ
25
30
40
50

Abb. 10

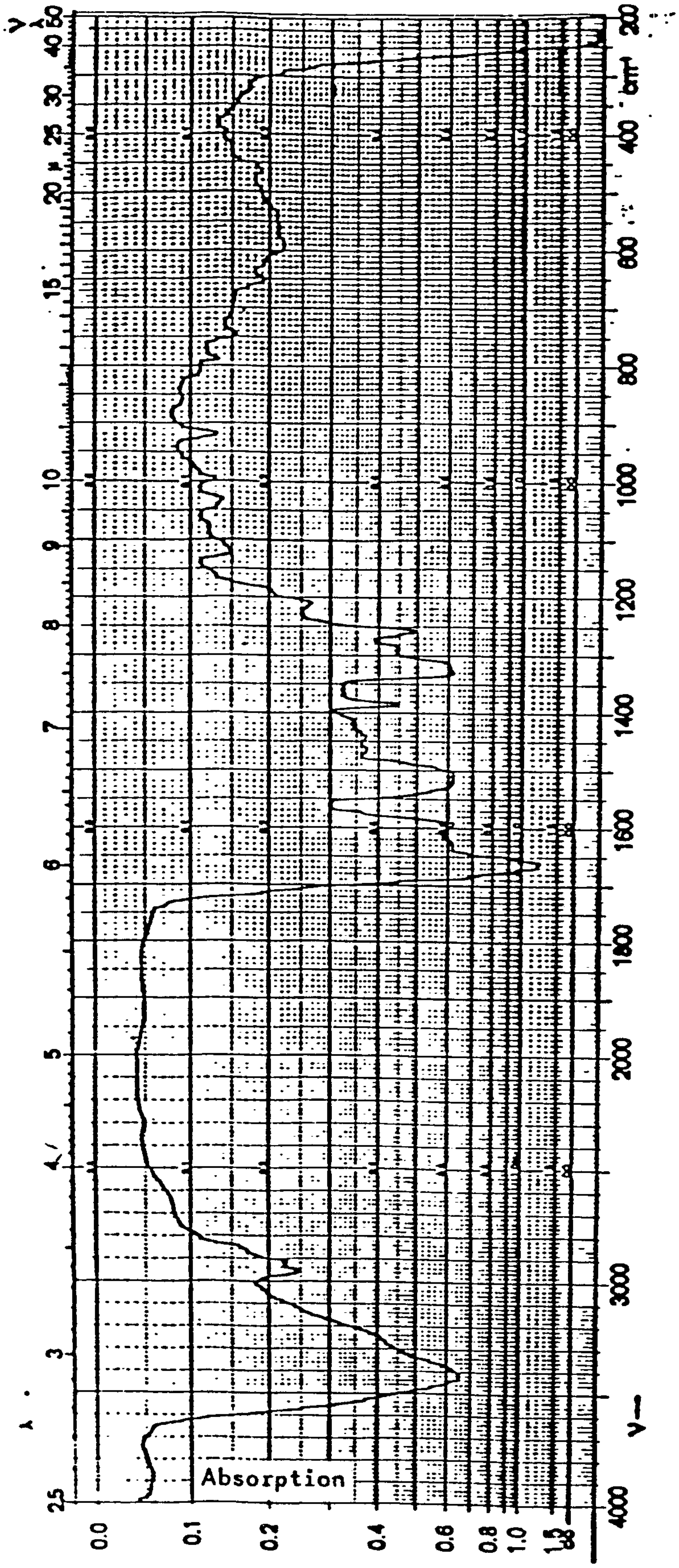
Absorption
0.0
0.1
0.2
0.4
0.6
0.8
1.0
1.5
∞
4000
3000
2000
1800
1600
1400
1200
1000
800
600
400
200
cm⁻¹
2.5
3
4
5
6
7
8
9
10
15
20 μ
25
30
40
50

65